# EUROPEAN PATENT SPECIFICATION

(11) **EP 0 528 447 B1**
(45) Date of publication and mention of the grant of the patent: **29.01.1997**
(21) Application number: 92117045.2
(22) Date of filing: 16.10.1985
(51) Int. Cl.: A61F 2/46

(54) **Device for producing bone cement for fixing protheses**
Vorrichtung zum Produzieren von Knochenzement zur Fixierung von Prothesen
Dispositif pour la production de ciment pour os pour fixer des prothèses

(30) Priority: 19.10.1984 SE 8405232
(43) Date of publication of application: 24.02.1993
(62) Divisional of application: 85113151.6
(73) Proprietor: MIT AB, S-275 37 Sjöbo (SE)
(72) Inventor: Lidgren, Lars Ake Alvar, S-227 31 Lund (SE)
(74) Representative: Wagner, Karl Heinz

(56) References cited:
- WO-A-84/03830
- US-A- 4 015 945
- US-A- 4 277 184
- US-A- 5 328 262
- Orthopedics, Nov.1987, Vol.10, Nr. 11, Alkire et al, "High vacuum as a method of reducing porosity of polymethylmethacrylate"

## Description

The present invention relates to a device for carrying out a method for producing bone cement for fixing protheses, whereby the bone cement is manufactured by mixing various substances with each other to provide a setting mass which in set condition is adapted to fix protheses at surrounding bone tissue, whereby the device comprises a feed device with a movable piston, the mixture being applied to the bone tissue by means of said feed device from which the mixture is pressed out by means of said movable piston, whereby the feed device is provided with a cylinder forming a first and a second end portion and a space, whereby the device further comprises an agitator for mixing said various substances in said space, said agitator being connectable to the feed device at the second end portion of the cylinder, whereby the first end portion of the cylinder is provided with said piston, whereby a discharge tube is connectable to the feed device at the second end of the cylinder after removing the agitator therefrom, and whereby the mixture is dischargeable from the space of the feed device by means of a discharge device adapted to press the piston towards said discharge tube.

A great problem when producing bone cement is that air is introduced during mixing of the various components or substances required for the production of bone cement. Because of this introduction or addition of air into the mixture, the bone cement becomes porous and thus, looses some of the desired properties.

In order to eliminate this problem, one has discussed to carry out mixing of the various substances at such a great vacuum that the introduction of air into the mixture is reduced. However, there is no appropriate device for this purpose.

It is already known to use a vacuum for sucking out poisonous or toxic gases generated during mixing of the substances for producing bone cement. According to US patent specification 4 277 184 this can be done by connecting a bone cement mixer and dispenser to a vacuum source for generating a vacuum in the mixing space or mixing chamber of said mixer and dispenser.

The mixer and dispenser according to the US-A-4 277 184 has a cylinder wherein mixing of the bone cement is carried through. Mixing occurs by means of a mixing member which is connected to the cylinder at the upper end thereof. The plunger for the mixed bone cement is connected to the cylinder at the same upper end and with this plunger the bone cement is discharged through the lower end of the cylinder via an extension tube. Additionally, a vacuum tube for connecting the cylinder to a vacuum source can be connected to said upper end of the cylinder.

Since the vacuum source is connectable to the cylinder at said upper end thereof, there is a risk that the plunger closes the port to the vacuum source, which means that there is a risk that said vacuum source can not generate a vacuum of any kind in the cylinder.

The object of the present invention has been to minimize the introduction or addition of air into the mixture during manufacture of bone cement and this is arrived at according to the invention by providing the initially defined device with the characterizing features of subsequent claim 1.

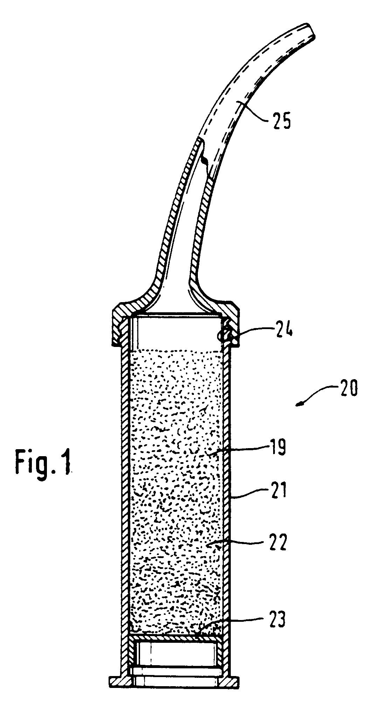

The invention will be further described below with reference to the accompanying drawings, in which
fig. 1 is a section through a feed device according to the present invention for mixing and delivering bone cement to bone tissue,

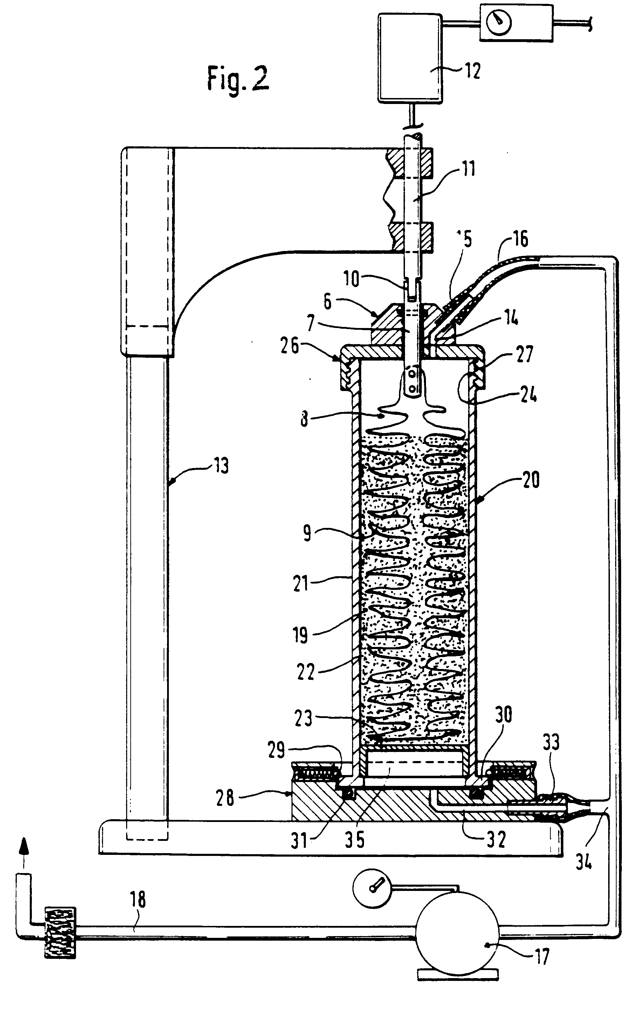

fig. 2 is a section through another device according to the present invention for mixing and delivering bone cement to bone tissue; and

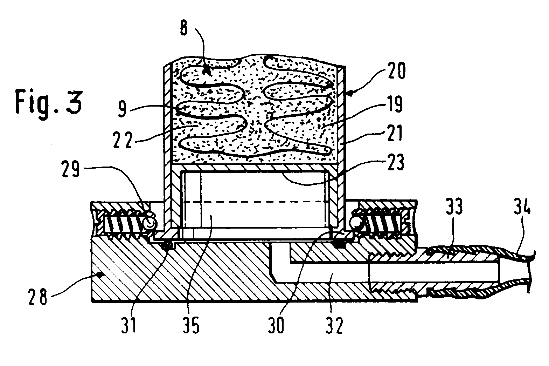

fig. 3 is a section through the lower portion of the feed device of fig. 2.

The feed devices 20 shown in the drawings are adapted to feed or apply bone cement to the bone onto which a prothesis shall be fixed. Each feed device 20 comprises a cylinder 21 with a space 22 for a bone cement mixture. Each feed device 20 further comprises a movable piston 23 which is provided at a first end portion thereof and adapted to press the mixture 19 out of the feed device 20 through a discharge tube 25 provided at the other end portion of said feed device. The piston 23 is displaceable by means of a discharge device (not shown) of prior art type. Each feed device 20 also comprises a threaded end portion 24 onto which the discharge tube 25 can be screwed.

When the various substances for obtaining or manufacturing the bone cement mixture 19 have been fed or supplied to the mixing space 22 in the feed device 20, said substances are mixed by means of a rotatable agitator 8. This agitator 8 is connectable to said other end portion of the feed device 20, e.g. while a shaft 7 forming part of the agitator 8 is rotatably mounted in a cap 26 or in a bearing or mounting member 6 provided on said cap. The cap 26 may have threads 27 such that it can be screwed onto the threaded end portion 24 of the cylinder 21. The agitator 8 further comprises an agitator blade 9 which is mounted on the shaft 7 and provided in the mixing space 22. The shaft 7 of the agitator 8 preferably has a recess 10 for permitting connection of the shaft 7 to an output shaft 11 of a driving motor 12 for operating the agitator 8. The driving motor 12 is mounted on a frame 13, onto which the feed device 20 may be positioned for carrying out the mixing procedure.

The space 22 of the cylinder 21 is connectable to a vacuum pump 17, which is adapted to generate such a vacuum, preferably a vacuum of 0,05-0,5 bar absolute pressure, in the space 22 during mixing of the various substances therein that addition of air into the mixture 19 is minimized. Moreover, the vacuum pump 17 is preferably connectable to the space 22 when mixing is carried out in an operation room wherein the bone is operated. The vacuum pump 17 is preferably connectable to the second end portion of the cylinder.

The cap 26 and its mounting member 6 has preferably a passage 14 and a connecting nipple 15 is provided on the mounting member 6, the interior of said nipple defining an extension of said passage 14. A hose 16 to the vacuum pump 17 is connectable to the connecting nipple 15 and said pump 17 is adapted to obtain the vacuum in the mixing space 22 by sucking out air therefrom through the passage 14, connecting nipple 15 and hose 16. The vacuum pump 17 is adapted to lead out air and gases from the mixing space 22 through an outlet conduit 18 and also from the operating room wherein the mixing is carried out.

For the manufacture of bone cement, two or more suitable substances are placed in the space 22 of the feed device 20. Of these substances, one substance is a liquid, substantially including monomethylmethacrylate, and another substance a powder, substantially consisting of polymethylmethacrylate. By mixing said substances and eventual additional substances of suitable type, a mixture 19 is obtained which is set to an acrylate-based bone cement.

In order to obtain said mixture 19, said liquid is poured into the space 22 of the feed device 20. Eventually, the liquid may be cooled before or after supplying it to the mixing space 22 in order to facilitate the mixing procedure and/or improve the properties of the final product. After supplying the liquid to the mixing space 22, the powder is added to said liquid. The cap 26 is placed on the feed device 20 and the agitator shaft 7 is connected to the drive shaft 11. Thereafter, the hose 16 from the vacuum pump 17 is connected to the connecting nipple 15 and the pump is activated. The vacuum pump 17 is operated a certain time, e.g. at least 5 seconds. preferably at least 10 seconds, until a vacuum of at least 70%, preferably 90-98%, is generated in the mixing space 22. It is generally suitable to generate by means of the vacuum pump 17 a vacuum (negative pressure) of 0,05-0,5 bar absolute pressure and carry out the mixing at this vacuum. When the desired vacuum is generated, the agitator 8 is activated to rotate and because of this stirring, the substances in the space 22 will mix under vacuum to a suitable mixture 19. While the mixing procedure is carried out under vacuum, introduction or addition of air into the mixture 19 during stirring is minimized. A minimum stirring time is required for efficient stirring of the mixture 19. This is e.g. 30-90 seconds, preferably 30-60 seconds. It may be advantageous to carry out the mixing procedure at a temperature of 0-21°C in the mixing space 22. After the required mixing, the vacuum is abolished and the mixture 19 obtained in the mixing space 22 is transferred to that cavity in the bone tissue wherein the prothesis shall be fixed. Thereafter, the prothesis is inserted into the mixture 19 in the bone tissue and placed in the desired position, whereafter the mixture is setting. After setting, the bone cement fix the prothesis in its position.

To facilitate mixing of the mixture 19 in the space 22 of the feed device 20, the frame 13 is preferably provided with a holding device 28 adapted to hold the cylinder 21 of the feed device 20 during the mixing procedure with the agitator 8. The holding device 28 comprises spring loaded balls 29 which can snap over a lower collar 30 on the cylinder 21. The underside of the lower collar 30 engages the holding device 28 through a sealing ring 31. The holding device 28 has a passage 32 opening into a connecting nipple 33. A branch 34 of the hose 16 to the vacuum pump 17 is connectable to said nipple 33.

Vacuum is generated in the space 22 on one side of the piston 23 as well as in a space 35 beneath the piston. This means that the piston 23 will not be sucked upwards by a vacuum in the space 22, since there is a corresponding vacuum on the other side of the piston 23.

The invention is not limited to the described device, but may vary within the scope of the following claims.

## Claims

1. Device for carrying out a method for producing bone cement for fixing protheses,
whereby the bone cement is manufactured by mixing various substances with each other to provide a setting mass which in set condition is adapted to fix protheses at surrounding bone tissue,
whereby the device comprises a feed device (20) with a movable piston (23), the mixture being applied to the bone tissue by means of said feed device (20) from which the mixture (19) is pressed out by means of said movable piston (23),
whereby the feed device (20) is provided with a cylinder (21) forming a first and a second end portion and a space (22),
whereby the device further comprises an agitator (8) for mixing said various substances in said space (22), said agitator being connectable to the feed device (20) at the second end portion of the cylinder (21,
whereby the first end portion of the cylinder (21) is provided with said piston (23),
whereby a discharge tube (25) is connectable to the feed device at the second end of the cylinder (21) after removing the agitator (8) therefrom, and
whereby the mixture (19) is dischargeable from the space (22) of the feed device (20) by means of a discharge device adapted to press the piston (23) towards said discharge tube (25),
**characterized in**
that the device further comprises a vacuum source connectable to the feed device (20) for generating a vacuum of 0,05 - 0,5 bar in said space (22) to carry out the mixing of the various substances with the agitator (8) at this vacuum for minimizing the porosity of the mixture (19) by minimizing introduction or addition of air into the mixture (19), and
that the vacuum source for generating said vacuum is a vacuum pump (17) connectable to the feed device (20) at the same second end of the cylinder (21) to which the agitator (8) and the discharge tube (25) are connectable.

2. Device according to claim 1, **characterized in** that the agitator (8) and the vacuum pump (17) are connectable to the second end portion of the cylinder (21) through a cap (26).

3. Device according to claim 2, **characterized in** that the second end portion of the cylinder (21) is provided with threads (27) for connecting threaded portions of the discharge tube (25) and the cap (26) to said second end portion.

4. Device according to any preceding claim, whereby the feed device (20) comprises a displaceable piston (23) which is adapted to be moved into a space (22) with the mixture (19) for pressing out the mixture therefrom and whereby mixing of said mixture occurs in said space (22) while said space is connected to the vacuum source for generating said vacuum in said space (22), **characterized in** that the vacuum source is a vacuum pump (17) which is provided also to subject a space (35) on the outside of the piston (23) to said vacuum while it at the same time generates said vacuum in the space (22) within the piston, whereby the space (35) is defined by the outer side of the piston and by a holding device (28) for holding the feed device (20) during the mixing procedure.

## Patentansprüche

1. Vorrichtung zum Produzieren von Knochenzement zur Fixierung von Prothesen,
wobei der Knochenzement durch Mischen verschiedener Substanzen hergestellt wird, so daß eine abbindende Masse erzielt wird, die unter gegebenen Bedingungen geeignet ist, Prothesen im umgebenden Knochengewebe zu fixieren,
wobei die Vorrichtung eine Zuführvorrichtung (20) mit einem beweglichen Kolben (23) aufweist und die Mischung auf das Knochengewebe mit Hilfe der genannten Zuführvorrichtung (29) aufgetragen wird, aus der die Mischung (19) mit Hilfe des beweglichen Kolbens (23) herausgepreßt wird,
wobei die Zuführvorrichtung (29) mit einem Zylinder (21) ausgerüstet ist, der einen ersten und einen zweiten Endbereich und einen Raum (22) bildet,
wobei die Vorrichtung außerdem ein Rührwerk (8) zum Mischen der genannten verschiedenen Substanzen im genannten Raum (22) aufweist, wobei das genannte Rührwerk am zweiten Ende des Zylinders (21) an die Zuführvorrichtung (20) angeschlossen werden kann,
wobei sich im ersten Endbereich des Zylinders (21) der genannte Kolben (23) befindet,
wobei eine Entladungsröhre (25) am zweiten Endbereich des Zylinders (21) an die Zuführvorrichtung angeschlossen werden kann, nachdem das Rührwerk (8) entfernt wurde, und
wobei die Mischung (19) aus dem Raum (22) der Zuführvorrichtung (20) mit Hilfe einer Entladungsvorrichtung entladen werden kann, die geeignet ist, den Kolben (23) zu der genannten Entladungsröhre hin zu drücken,
**gekennzeichnet dadurch**,
daß die Vorrichtung weiter eine Vakuumquelle aufweist, die an die Zuführvorrichtung (20) angeschlossen werden kann, so daß im genannten Raum ein Vakuum von 0,05 - 0,5 bar erzeugt wird, damit die verschiedenen Substanzen mit dem Rührwerk (8) bei diesem Vakuum gemischt werden, so daß die Porosität der Mischung (19) durch Minimierung der eindringenden oder eingebrachten Luft in die Mischung möglichst klein gehalten wird, und
daß die Vakuumquelle zur Erzeugung des genannten Vakuums eine Vakuumpumpe (17) ist, die an die Zuführvorrichtung (20) am selben zweiten Endbereich des Zylinders (21) angeschlossen werden kann, an die das Rührwerk (8) und die Entladungsröhre angeschlossen werden können.

2. Vorrichtung nach Anspruch 1, **gekennzeichnet dadurch**, daß das Rührwerk (8) und die Vakuumpumpe (17) durch einen Deckel (26) an den zweiten Endbereich des Zylinders (21) angeschlossen werden.

3. Vorrichtung nach Anspruch 2, **gekennzeichnet dadurch**, daß der zweite Endbereich des Zylinders (21) mit Gewinden (27) zum Anschluß der mit Gewinde versehenen Teile der Entladungsröhre (25) und des Deckels (26) an den genannten Endbereich ausgerüstet ist.

4. Vorrichtung nach einem der vorstehenden Ansprüche, wobei die Zuführvorrichtung (20) einen verschiebbaren Kolben (23) aufweist, der geeignet ist, in einen Raum (22) mit der Mischung (19) hineinbewegt zu werden und die Mischung daraus hinauszupressen, und wobei das Mischen der genannten Mischung in denm Raum (22) stattfindet, während der Raum (22) an die Vakuumquelle angeschlossen ist, die in dem Raum (22) das genannte Vakuum erzeugt, **gekennzeichnet dadurch**, daß die Vakuumquelle eine Vakuumpumpe (17) ist, die dazu dient, auch in einem Raum (35) außerhalb des Kolbens (23) das genannte Vakuum zu erzeugen, während sie gleichzeitig das genannte Vakuum im Raum (22) innerhalb des Kolbens erzeugt, wobei der Raum (35) durch die Außenseite des Kolbens und eine Haltevorrichtung (28) zum Halten der Zuführvorrichtung (20) während des Mischvorgangs definiert ist.

## Revendications

1. Dispositif pour appliquer une méthode de production de ciment pour os pour fixer des prothèses,
le ciment pour os résultant de la mixtion de diverses substances en vue d'obtenir une masse durcissante permettant, durcie, de fixer des prothèses au tissu osseux circonvoisin,
le dispositif comprenant un dispositif d'alimentation (20) à piston mobile (23), le mélange étant appliqué sur le tissu osseux à l'aide dudit dispositif d'alimentation (20) duquel le mélange (19) est exprimé au moyen dudit piston mobile (23),
le dispositif d'alimentation (20) étant pourvu d'un cylindre (21) formé d'une première et d'une seconde extrémités et d'une enceinte (22),
le dispositif comprenant en outre un agitateur (8) pour mélanger lesdites diverses substances dans ladite enceinte (22), ledit agitateur pouvant être raccordé au dispositif d'alimentation (20) par la seconde extrémité du cylindre (21),
la première extrémité du cylindre étant pourvue dudit piston (23),
un tuyau d'évacuation (25) pouvant être raccordé au dispositif d'alimentation par la seconde extrémité du cylindre (21) après en avoir enlevé l'agitateur (8),
le mélange (19) pouvant être exprimé de l'enceinte (22) du dispositif d'alimentation (20) à l'aide d'un dispositif d'évacuation conçu pour pousser le piston (23) en direction dudit tuyau d'évacuation (25),
**caractérisé en ce que**
le dispositif comprend en outre une source de vide pouvant être raccordée au dispositif d'alimentation (20) dans le but de créer un vide de 0,05 à 0,5 bar dans ladite enceinte (22) pour effectuer la mixtion des diverses substances à l'aide de l'agitateur (8) dans ce vide afin de réduire autant que possible la porosité du mélange (19) en réduisant autant que possible l'adjonction d'air au mélange (19), et en ce que
la source de vide utilisée pour créer ledit vide est une pompe à vide (17) pouvant être raccordée au dispositif d'alimentation (20) par la même seconde extrémité du cylindre (21) à laquelle peuvent être raccordés l'agitateur (8) et le tuyau d'évacuation (25).

2. Dispositif selon la revendication 1, **caractérisé en ce que** l'agitateur (8) et la pompe à vide (17) peuvent être raccordés à la seconde extrémité du cylindre (21) à travers un capuchon (26).

3. Dispositif selon la revendication 2, **caractérisé en ce que** la seconde extrémité du cylindre (21) est pourvue de filets (27) pour pouvoir joindre les parties filetées du tuyau d'évacuation (25) et du capuchon (26) à ladite seconde extrémité.

4. Dispositif selon l'une quelconque des revendications précédentes, le dispositif d'alimentation (20) comprenant un piston mobile (23) conçu pour pénétrer dans une enceinte (22) contenant le mélange (19) et pour en exprimer ledit mélange, la mixtion dudit mélange ayant lieu dans ladite enceinte (22), laquelle enceinte étant en liaison avec la source de vide pour que soit créé ledit vide dans ladite enceinte (22), **caractérisé en ce que** la source de vide est une pompe à vide (17) laquelle étant également conçue pour soumettre un espace (35) à l'extérieur du piston (23) audit vide tout en créant ledit vide dans l'espace (22) à l'intérieur du piston, cet espace (35) étant défini par la face extérieure du piston et par un dispositif de fixation (28) pour immobiliser le dispositif d'alimentation (20) pendant la mixtion.
